# EUROPEAN PATENT SPECIFICATION

(11) **EP 2 022 388 B1**
(45) Date of publication and mention of the grant of the patent: **29.05.2013**
(21) Application number: 08011862.3
(22) Date of filing: 01.07.2008
(51) Int. Cl.: A61B 1/00, A61B 1/05, A61B 1/04, A61B 1/045, A61B 1/06

(54) **Endoscope apparatus**
Endoskopvorrichtung
Appareil d'endoscopie

(30) Priority: 10.08.2007 JP 2007210003
(43) Date of publication of application: 11.02.2009
(73) Proprietor: Olympus Medical Systems Corp., Tokyo 151-0072 (JP)
(72) Inventor: Takahashi, Tomoya, Tokyo, 151-0072 (JP)
(74) Representative: von Hellfeld, Axel

(56) References cited:
- EP-A- 1 712 177
- EP-A- 1 779 768
- US-A- 4 884 133
- US-A- 5 408 263
- US-A- 5 967 969
- US-A1- 2002 156 349

## Description

### BACKGROUND OF THE INVENTION

### 1. Field of the Invention

The present invention relates to an endoscope apparatus and, more particularly, to an endoscope apparatus capable of arbitrarily setting an illumination time for a light source device which illuminates an object to be observed by an image pickup portion with illumination light.

### 2. Description of the Related Art

Medical endoscope apparatuses have now been commonly and widely used. The endoscope apparatuses observe an alimentary canal including an esophagus, stomach, small intestine, and large intestine or a trachea leading to lungs by inserting an elongated insertion portion into a body cavity. The endoscope apparatuses can perform various treatment processes using a treatment instrument inserted in a treatment instrument channel as needed. Among the endoscope apparatuses, an electronic endoscope apparatus having a solid-state image pickup device such as a charge coupled device (CCD) as an image pickup portion is in wide use. The electronic endoscope apparatus is capable of displaying a moving image on a color monitor in real time. The electronic endoscope apparatus thus causes less fatigue on a surgeon which manipulates an endoscope.

The electronic endoscope apparatus has an electronic endoscope incorporating the CCD at a distal end portion of an insertion portion, a processor device which performs video signal processing, and a light source device which supplies illumination light. The electronic endoscope can be detachably connected to the processor device and light source device.

Accordingly, in the electronic endoscope apparatus, various types of electronic endoscopes can be used in combination with one processor device and one light source device. One of CCDs different in pixel count and the like is mounted in the electronic endoscope depending on a part into which the electronic endoscope is to be inserted or an intended use. A charge readout time varies depending on a type of the CCD.

As the CCD used in the electronic endoscope, one whose charge accumulating portion also serves as a charge transfer channel may be adopted to reduce a device size. In the case, the electronic endoscope apparatus needs to prevent an image of a subject from being formed at the CCD by cutting off illumination light applied to the subject during a charge readout period for the CCD.

For the reason, a frame sequential type electronic endoscope apparatus which sequentially applies red, blue, and green light beams and picks up an image rotates a filter plate having a light-shielding portion provided between filters of the respective colors. With the configuration, the frame sequential type electronic endoscope apparatus cuts off light during a light-shielding period. Duration of a light-shielding period is set to suit a CCD with a longest charge readout time.

In such a frame sequential type electronic endoscope apparatus, a charge readout time, i.e., an image pickup time (permissible exposure time) varies depending on a type of a solid-state image pickup device, such as a charge coupled device (CCD), serving as an image pickup portion used. For the reason, in an electronic endoscope apparatus, a required light-shielding period, i.e., a required illumination time for a light source device (a light emission time within a permissible CCD exposure time range) varies depending on a type of a solid-state image pickup device.

However, in a conventional frame sequential type electronic endoscope apparatus, an illumination time for a light source device (a light emission time within a permissible CCD exposure time range) is constant, whichever electronic endoscope is used and regardless of a permissible CCD exposure time for the electronic endoscope.

For the reason, in the conventional frame sequential type electronic endoscope apparatus, even if a CCD image pickup time (the permissible exposure time) is longer than the illumination time, a required amount of light is not obtained due to a light-shielding period. This is inconvenient for increasing brightness of the electronic endoscope apparatus itself.

Since the illumination time for the light source device is constant, even if efforts, such as increasing the number of light guides of the electronic endoscope and increasing an f-number of an illumination lens of an illumination optical system, are made to increase brightness of the electronic endoscope apparatus, a diameter of the electronic endoscope increases or design of the illumination optical system becomes complicated. This may interfere with an improvement in specifications of a new electronic endoscope.

If the CCD of the electronic endoscope is a high-resolution fast CCD, since the illumination time for the light source device is constant, the amount of light cannot be limited with a diaphragm at the time of near-point observation. This may result in halation such as high luminance or whiteout.

Appropriate values of a wavelength of emitted light and a luminance balance vary depending on an observation mode such as a special light observation mode and a type of a light source of the light source device. Since the illumination time for the light source device is constant, it is difficult to control the appropriate values of the wavelength of emitted light and the luminance balance.

In other words, it is desired that the conventional frame sequential type electronic endoscope apparatus be configured to be capable of changing the illumination time for the light source device depending on a type of the electronic endoscope whichever type of electronic endoscope is used.

For example, Japanese Patent Application Laid-Open Publication No. 2002-119468 discloses a technique related to an endoscope apparatus which has a light modulating device for controlling whether to let illumination light from a light source lamp come incident on a light transmitting means side provided in an optical path of the light source lamp, includes a CCD type determining device storing CCD type determination information for determining a type of a CCD serving as an image pickup portion of an electronic endoscope, and sets an illumination time for a light source device by controlling the light modulating device on the basis of the CCD type determination information from the CCD type determining element.

In the conventional endoscope apparatus disclosed in Japanese Patent Application Laid-Open Publication No. 2002-119468, only CCD type determination information from the CCD type determining element of the electronic endoscope is used as information for setting the illumination time for the light source device, and the illumination time for the light source device is set on a video processor side on the basis of the CCD type determination information.

US 5967969 discloses an endoscope apparatus including a peripheral device having a light source and a connecting device, wherein the illumination duration (i.e., usage time) of the light source is measured and stored in the peripheral device such that the total usage hours and the number of times that each endoscope is used are recorded to ease maintenance requirements and improve operating quality. An indicating device is used to display the stored illumination duration independently of the connection between the endoscope and the peripheral device.

However, an illumination time for a light source device suitable for a CCD serving as an image pickup portion of an electronic endoscope largely depends not only on a CCD but also on components of an optical system, such as an objective lens and the number of light guides. For the reason, a conventional endoscope apparatus can set an illumination time for a light source device only when which type of CCD is used as an image pickup portion is known beforehand and cannot set the illumination time for the light source device on the basis of a CCD and components of an optical system as described above.

The present invention has been made in consideration of the above-described circumstances, and has its object to provide an endoscope apparatus capable of arbitrarily setting an illumination time for a light source device depending on a type of an electronic endoscope whichever type of electronic endoscope is used.

### SUMMARY OF THE INVENTION

These problems are solved by an endoscope apparatus according to claim 1.

An endoscope apparatus according to the present invention includes a connection portion to which medical equipment having an image pickup portion can be connected, a light source portion which applies illumination light to an object to be observed by the image pickup portion, an illumination time changing portion which changes an illumination time for illumination light from the light source portion, a storage portion provided in the medical equipment and storing illumination time information for illumination light suitable for the medical equipment, and a control portion which, upon connection of the medical equipment, reads out the illumination time information stored in the storage portion and controls the illumination time changing portion such that the illumination time for the light source portion is consistent with the read-out illumination time information.

### BRIEF DESCRIPTION OF THE DRAWINGS

Fig. 1 is a configuration diagram showing an endoscope apparatus according to the present invention;
Fig. 2A is a chart showing timing for an R/G/B identification signal outputted from a utilization factor controlling portion of a light source device to a video signal processing portion;
Fig. 2B is a chart showing timing for a CV-light source synchronizing signal generated by a timing generator of a video processor;
Fig. 2C is a chart showing a default for illumination time information stored in a storage portion (not shown) in the utilization factor controlling portion;
Fig. 2D is a chart showing loaded illumination time information;
Fig. 2E is a chart showing CCD exposure periods (image pickup periods);
Fig. 3A is a chart showing timing for an R/G/B identification signal outputted from a utilization factor controlling portion of a light source device to a video signal processing portion in a second embodiment;
Fig. 3B is a chart showing timing for a CV-light source synchronizing signal generated by a timing generator of a video processor in the second embodiment;
Fig. 3C is a chart showing timing for turning on a stroboscopic light source in the second embodiment; and
Fig. 3D is a chart showing CCD exposure periods (image pickup periods) in the second embodiment.

### DETAILED DESCRIPTION OF THE PREFERRED EMBODIMENTS

Embodiments of the present invention will be described below with reference to the drawings.

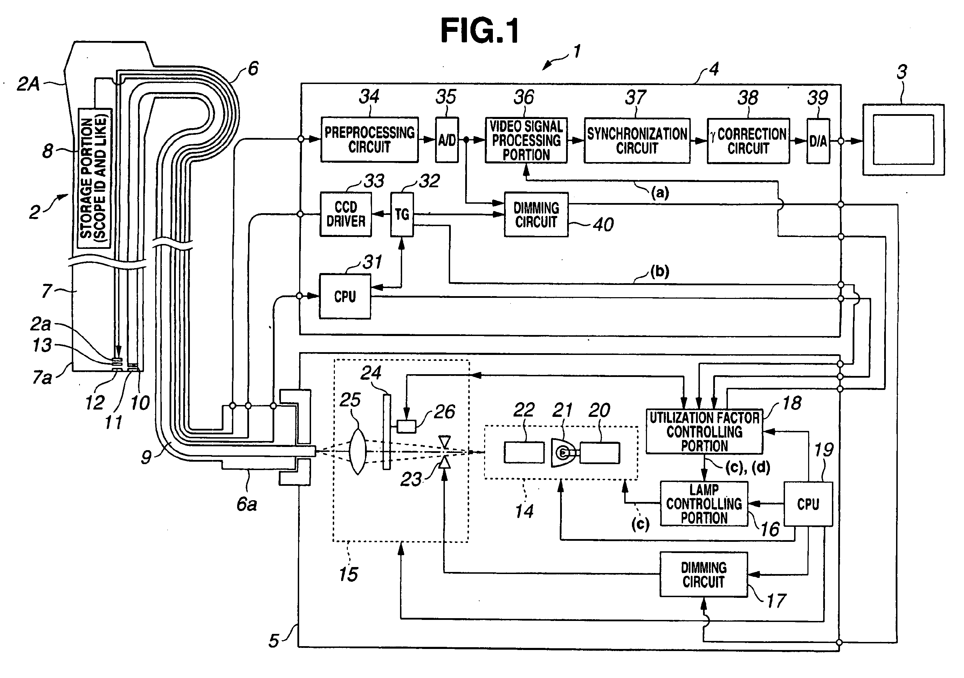

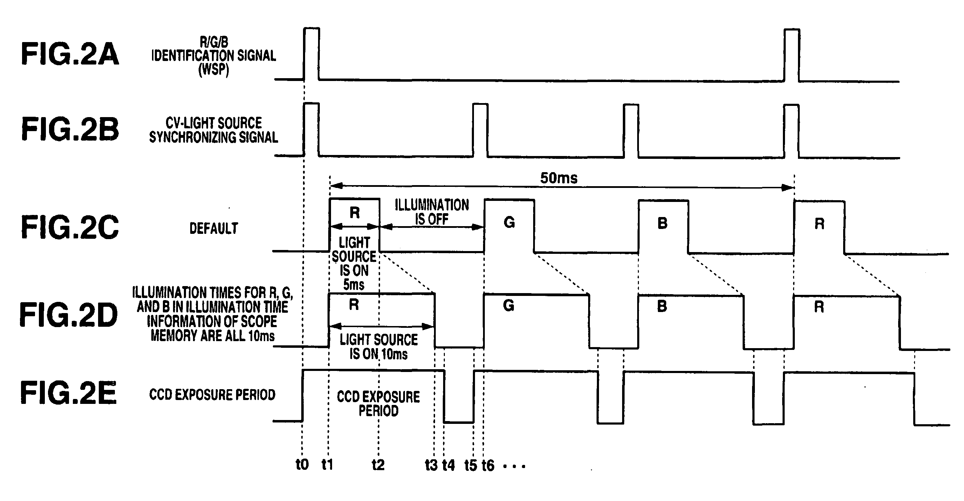

Figs. 1 to 2E relate to the present invention. Fig. 1 is a configuration diagram showing an endoscope apparatus according to the first embodiment. Fig. 2A is a chart showing timing for an R/G/B identification signal outputted from a utilization factor controlling portion of a light source device to a video signal processing portion. Fig. 2B is a chart showing timing for a CV-light source synchronizing signal generated by a timing generator of a video processor. Fig. 2C is a chart showing a default for illumination time information stored in a storage portion (not shown) in the utilization factor controlling portion. Fig. 2D is a chart showing loaded illumination time information. Fig. 2E is a chart showing CCD exposure periods (image pickup periods).

As shown in Fig. 1, an endoscope apparatus 1 according to the invention is configured to have an electronic endoscope (hereinafter simply referred to as the endoscope) 2 incorporating a CCD 2a as an image pickup portion, a video processor 4 which processes image pickup signals derived from an image picked up by the CCD 2a of the endoscope 2 and displays an observation image on a monitor 3, and a light source device 5 which supplies illumination light to the endoscope 2. Note that the endoscope 2 constitutes the above-described medical equipment.

The endoscope 2 is configured to include an operation portion 2A on a proximal end side of an elongated insertion portion 7. The endoscope 2 is detachably connected to the light source device 5 and video processor 4 through a connector portion 6a provided at an end of a universal cable 6 which extends from a side of the operation portion 2A and in which a light guide (to be described later) and the like are inserted. Note that the connector portion 6a constitutes the above-described connection portion.

The endoscope 2 incorporates the CCD 2a at a distal end portion 7a of the insertion portion 7.

The endoscope 2 is configured such that a storage portion 8, storing illumination time information for setting an illumination time for the light source device 5 which is suitable for the CCD 2a of the endoscope 2 and a component of an optical system such as an objective lens, is provided in the operation portion 2A.

Note that the storage portion 8 may be provided at the connector portion 6a or in the insertion portion 7. The storage portion 8 stores pieces of information such as a scope ID of the endoscope 2, to which the storage portion 8 belongs, in addition to the illumination time information.

The illumination time information is illumination time information including respective illumination times for R light, G light, and B light (e.g., R: 10 msec, G: 10 msec, B: 10 msec).

A light guide 9 capable of transmitting a subject image from the distal end portion 7a of the insertion portion 7 to the connector portion 6a of the universal cable 6 is inserted in the endoscope 2. Illumination light from the light source device 5, having been transmitted from the light guide 9, shines on a subject through an illumination lens 10 and an illumination cover glass 11. The endoscope 2 is configured such that a subject image captured through the illumination cover glass 11 is imaged on an image pickup surface of the CCD 2a by an objective optical system 13.

The endoscope 2 with the above-described configuration is detachably connected to the light source device 5 and video processor 4 through the connector portion 6a, as described above.

The video processor 4 is configured to have a CPU 31 which reads out the illumination time information and the pieces of information including the scope ID stored in the storage portion 8 of the endoscope 2 and transmits the pieces of information to the light source device 5, a timing generator (hereinafter referred to as the TG) 32 which provides timing for readout from the CCD 2a incorporated in the endoscope 2 and supplies a processing timing signal (e.g., a video processor-light source synchronizing signal shown in Fig. 2B) synchronized with the timing for readout, in accordance with a charge readout time detected by the CPU 31, a CCD driver 33 which drives the CCD 2a by the readout signal generated by the TG 32, a preprocessing circuit 34 which subjects an image pickup signal from the CCD 2a to CDS (correlated double sampling) and the like, an A/D converter circuit 35 which converts an image pickup signal outputted from the preprocessing circuit 34 from an analog signal into a digital signal, a video signal processing portion 36 which signal-processes an image pickup signal digitized by the A/D converter circuit 35 into a video signal, a synchronization circuit 37 which performs synchronization of frame sequential images by sequentially storing video signals derived from signal processing by the video signal processing portion 36 in a plurality of synchronization memories (not shown) and simultaneously reading out the stored video signals, a γ correction circuit 38 which subjects a video signal from the synchronization circuit 37 to γ correction, and a D/A converter circuit 39 which converts a digital video signal after γ correction by the γ correction circuit 38 into an analog signal.

The video processor 4 also has a dimming circuit 40 which generates a signal for a diaphragm 23 of the light source device 5 from an image pickup signal digitized by the A/D converter circuit 35 and outputs the signal to a dimming circuit 17 of the light source device 5, on the basis of the processing timing provided by the TG 32.

The TG 32 of the video processor 4 outputs the CV-light source synchronizing signal (see Fig. 2B) synchronized with the timing for readout from the CCD 2a provided by the TG 32 to a utilization factor controlling portion 18 of the light source device 5.

An R/G/B identification signal (see Fig. 2A) from the utilization factor controlling portion 18 of the light source device 5 is supplied to the video signal processing portion 36. The video signal processing portion 36 signal-processes a digitized image pickup signal into a video signal on the basis of timing provided by the supplied R/G/B identification signal.

A configuration of the light source device 5 will now be described.

The light source device 5 is configured to have a light source portion 14 which generates light for supplying illumination light to the endoscope 2 and emits illumination light, an illumination time variator unit 15 for changing illumination times for illumination light from the light source portion 14, a lamp controlling portion 16 which controls the light source portion 14, the dimming circuit 17 which controls the diaphragm 23 in the illumination time variator unit 15, the utilization factor controlling portion 18, to which illumination time information is supplied from the CPU 31 of the video processor 4 and which controls the illumination time variator unit 15 such that each illumination time is set on the basis of the illumination time information, and a CPU 19 which controls the entire light source device 5 including the light source portion 14, illumination time variator unit 15, lamp controlling portion 16, dimming circuit 17, and utilization factor controlling portion 18.

Note that the illumination time variator unit 15 constitutes the above-described illumination time changing portion. Also note that the lamp controlling portion 16, dimming circuit 17, utilization factor controlling portion 18, and CPU 19 constitute the above-described control portion.

The light source portion 14 is configured to have a light source lamp 21, such as a xenon lamp, which emits light for supplying illumination light to the endoscope 2, a driver 20 for driving the light source lamp 21, and an integrator 22 which uniformizes light emitted from the light source lamp 21.

The illumination time variator unit 15 is composed of the illumination light diaphragm (hereinafter simply referred to as the diaphragm) 23, which limits the amount of irradiation light uniformized by the integrator 22, a rotating filter plate 24 which transmits light beams having wavelengths in red, green, and blue ranges of irradiation light whose amount is limited by the diaphragm 23, and a condenser lens 25 which condenses light beams having wavelengths in red, green, and blue ranges, having passed through the rotating filter plate 24, on an incident end surface of the light guide 9.

Although not shown, an R transmission portion, a G transmission portion, and a B transmission portion which respectively transmit light beams having wavelengths in red, green, and blue ranges are arranged in the rotating filter plate 24. The rotating filter plate 24 is rotationally driven by a motor 26. Note that, although not shown, one chopper (light-shielding plate) is provided in the rotating filter plate 24, and the rotating filter plate 24 and chopper are phase-controlled while being rotationally synchronized with each other under control of the motor 26 by the utilization factor controlling portion 18. That is, phase control of the rotating filter plate 24 and chopper allows illumination time change.

In the endoscope apparatus 1 according to the present invention, the illumination time information stored in the storage portion 8 of the endoscope 2 is loaded into the utilization factor controlling portion 18 in the light source device 5 through the CPU 31 of the video processor 4. The utilization factor controlling portion 18 controls the illumination time variator unit 15 such that each illumination time for the light source device 5 is consistent with the loaded illumination time information, thereby setting a utilization factor for the light source device 5.

Operation of the endoscope apparatus 1 with the above-described configuration will be described with reference to Figs. 2A to 2E.

Note that Fig. 2A shows the R/G/B identification signal outputted from the utilization factor controlling portion 18 of the light source device 5 to the video signal processing portion 36; Fig. 2B, the CV-light source synchronizing signal generated by the TG 32 of the video processor 4; Fig. 2C, a default for illumination time information stored in a storage portion (not shown) in the utilization factor controlling portion 18; Fig. 2D, the loaded illumination time information; and Fig. 2E, exposure periods (image pickup periods) for the CCD 2a.

First, a surgeon connects the connector portion 6a of the endoscope 2 to the light source device 5 and video processor 4, turns on power, and starts endoscopic examination.

The CPU 31 of the video processor 4 reads out the illumination time information and the pieces of information including the scope ID stored in the storage portion 8 of the endoscope 2 and outputs the read-out illumination time information to the utilization factor controlling portion 18 of the light source device 5.

The CPU 31 also detects a charge readout time for the CCD 2a from the pieces of information including the scope ID. The TG 32 provides the timing for readout from the CCD 2a and generates the CV-light source synchronizing signal synchronized with the timing for readout in accordance with the charge readout time detected by the CPU 31. The TG 32 outputs the CV-light source synchronizing signal to the dimming circuit 40 and the utilization factor controlling portion 18 of the light source device 5.

The storage portion (not shown) storing the default for illumination time information (preset time information) for the light source portion 14 and illumination time variator unit 15 is provided inside the utilization factor controlling portion 18 of the light source device 5.

For example, if a conventional endoscope without the storage portion 8 is connected, the utilization factor controlling portion 18 controls the illumination time variator unit 15 such that the illumination times for the light source device 5 are consistent with the default for illumination time information stored in the storage portion (e.g., a default including an illumination time of 5 msec each for R, G, and B as shown in Fig. 2C).

In contrast, if the endoscope 2 with the storage portion 8 is connected in the present invention, the utilization factor controlling portion 18 controls the illumination time variator unit 15 such that the illumination times for the light source device 5 are consistent with the loaded illumination time information, thereby setting the utilization factor for the light source device 5.

For example, assume that the loaded illumination time information includes an illumination time of 10 msec for R, an illumination time of 10 msec for G, and an illumination time of 10 msec for B, as shown in Fig. 2D. In the case, the utilization factor controlling portion 18 controls the illumination time variator unit 15 such that an illumination time from t1 to t3 when the R transmission portion of the RGB rotating filter plate 24 is located in an optical path is 10 msec. After that, the utilization factor controlling portion 18 similarly controls the illumination time variator unit 15 such that an illumination time for each of the G transmission portion and B transmission portion of the rotating filter plate 24 is 10 msec.

For a period for switching from the R transmission portion of the rotating filter plate to the G transmission portion in the optical path (a period from t3 to t6 in Fig. 2D), the utilization factor controlling portion 18 controls the illumination time variator unit 15 to block illumination light from the light source portion 14 with the chopper (not shown). After that, the utilization factor controlling portion 18 similarly controls the illumination time variator unit 15 for a period for switching from the G transmission portion of the rotating filter plate 24 and for a period for switching from the B transmission portion.

Note that, even if the illumination time information loaded from the endoscope 2 includes different pieces of illumination time information for R, G, and B, the utilization factor controlling portion 18 can provide control to set the utilization factor on the basis of the different pieces of illumination time information for R, G, and B.

If it is found through communication with the CPU 31 of the video processor 4 that the illumination time information is not stored in the storage portion 8 of the endoscope 2 or that the storage portion 8 itself is not present in the endoscope 2, the utilization factor controlling portion 18 sets the utilization factor such that the illumination times for the light source device 5 are consistent with the default (see Fig. 2C) for illumination time information stored in the storage portion (not shown).

Note that each illumination time based on the default or the illumination time information is set to fall within a CCD exposure period (image pickup period) shown in Fig. 2E.

When the illumination times for the light source device 5 are set in the above-described manner in the endoscope apparatus 1, the dimming circuit 40 of the video processor 4 and the dimming circuit 17 of the light source device 5 send a diaphragm control signal to the diaphragm 23 using an image pickup signal digitized by the A/D converter circuit 35 such that an image has appropriate brightness, on the basis of the processing timing provided by the TG 32.

The diaphragm 23 limits the amount of light emitted from the light source device 5 in response to the diaphragm control signal outputted from the dimming circuit 17 of the light source device 5 through the dimming circuit 40 of the video processor 4, thereby preventing excessive saturation in an image picked up by the CCD 2a.

The utilization factor controlling portion 18 controls the motor 26 of the rotating filter plate 24 on the basis of the CV-light source synchronizing signal (see Fig. 2B) generated by the TG 32 and the utilization factor set in the above-described manner such that the motor 26 is rotationally driven at a predetermined speed. In the rotating filter plate 24, the rotation of the motor 26 causes the R transmission portion, G transmission portion, and B transmission portion to be sequentially located in the optical path during a corresponding one of the set illumination times to transmit red, green, and blue light beams.

Light incident on the light guide 9 of the endoscope 2 is applied to a subject such as an alimentary canal from the distal end portion 7a of the insertion portion 7. Light scattered and reflected from the subject forms an image on the CCD 2a at the distal end portion 7a of the insertion portion 7.

The CCD 2a is driven by the CCD driver 33 in synchronism with the rotation of the rotating filter plate 24 on the basis of the timing signal from the TG 32. Image pickup signals corresponding to irradiation light beams, having passed through filters of the rotating filter plate 24 including the R transmission portion, G transmission portion, and B transmission portion, are sequentially outputted to the video processor 4.

The image pickup signals inputted to the video processor 4 are first inputted to the preprocessing circuit 34, are subjected to CDS (correlated double sampling) and the like, and are converted from analog signals into digital signals by the A/D converter circuit 35. The digitized image pickup signals are signal-processed into video signals by the video signal processing portion 36 and are sequentially stored in the plurality of synchronization memories (not shown). The synchronization circuit 37 simultaneously reads out the video signals sequentially stored in the plurality of synchronization memories and performs synchronization of frame sequential images. The frame sequential images after the synchronization are subjected to γ correction by the γ correction circuit 38, are converted into analog signals by the D/A converter circuit 39, and are outputted to the monitor 3.

According to the invention, since the illumination time information is directly loaded from the endoscope 2 to be connected, and the utilization factor for the light source device 5 is set such that the illumination times for the light source device 5 are set to be consistent with the loaded illumination time information, it is possible to arbitrarily set the illumination times for the light source device 5 depending on a type of the endoscope 2 whichever type of endoscope 2 is used.

Note that, an illumination time range and the illumination time information from the new endoscope 2 may be inconsistent with each other depending on a type of the illumination time variator unit 15 of the light source device 5. For the reason, if the illumination time information acquired by the utilization factor controlling portion 18 of the light source device 5 has values to which the illumination times cannot be changed (e.g., values outside the illumination time range), the utilization factor controlling portion 18 may set each illumination time to a value which is nearest to a corresponding one of the values of the illumination time information within the illumination time range.

More specifically, the utilization factor controlling portion 18 has comparison means for making a comparison to see whether the acquired illumination time information falls within the illumination time range corresponding to the illumination time variator unit 15 provided at the light source device and sets each illumination time to a value which is nearest to a corresponding value of the illumination time information within the illumination time range if a comparison result from the comparison means shows that the illumination time information does not fall within the illumination time range.

For example, if the illumination time information having a value of 13 msec is acquired from the endoscope 2 with respect to the light source device 5 with the illumination time range of 1 to 10 msec, the utilization factor controlling portion 18 controls the illumination time variator unit 15 such that each illumination time is set to 10 msec.

A storage portion storing respective pieces of illumination time information appropriate to types of the CCD 2a of the endoscope 2 may be provided in the utilization factor controlling portion 18 of the light source device 5.

In the case, the technique disclosed in Patent Document 1 as a prior art (an illumination time is set on the basis of CCD type determination information) is used. If illumination time information is not stored in the storage portion 8 of the endoscope 2 or if the storage portion 8 itself is not provided in the endoscope 2, the CPU 31 of the video processor 4 detects a type of the CCD 2a and outputs a result of determining the type of the CCD 2a to the utilization factor controlling portion 18 of the light source device 5.

The utilization factor controlling portion 18 of the light source device 5 may read out a piece of illumination time information appropriate to the type of the CCD 2a from the storage portion (not shown) on the basis of the determination result and set the piece of illumination time information.

In the invention, connection detecting means for detecting presence or absence of connection may be provided between the endoscope 2 and the video processor 4, between the video processor 4 and the light source device 5, and between the light source device 5 and the endoscope 2, a connection detection result from each connection detecting means may be sent to the utilization factor controlling portion 18, and the utilization factor controlling portion 18 may set the utilization factor such that the illumination times for the light source device 5 are consistent with the default for illumination time information (see Fig. 2C) stored in the storage portion (not shown) if the utilization factor controlling portion 18 determines from supplied connection detection results that all connections are not normally made.

In the endoscope apparatus 1 according to the invention, a normal light observation mode and a special light observation mode are different in brightness level necessary for each light wavelength. A brightness level varies depending on a type of the endoscope 2.

Accordingly, the illumination time information stored in the storage portion 8 of the endoscope 2 may be provided for each of observation modes including the normal light observation mode and special light observation mode. Note that, if a plurality of CCDs 2a are provided in the endoscope 2, respective pieces of illumination time information appropriate to observation modes are stored in the storage portion 8 for each of the plurality of CCDs 2a. Since illumination time information acquired by the utilization factor controlling portion 18 is illumination time information based on an observation mode, illumination times most suitable for the observation mode are obtained, and a necessary brightness level is obtained.

A wavelength and brightness of illumination light vary depending on a type of the light source lamp 21 in the light source portion 14. Accordingly, an illumination time correcting portion which corrects each illumination time to fall within an exposure period (image pickup period) for the CCD 2a depending on the type of the light source lamp 21 mounted at the light source device 5 on the basis of the illumination time information in the endoscope 2 may be provided in the utilization factor controlling portion 18 to perform illumination time correction processing. The configuration optimizes the amount of illumination light from the light source device 5 regardless of whether the light source lamp 21 is composed of a xenon lamp or another lamp.

For example, if the endoscope 2 with a different observation mode is connected, the illumination time correcting portion corrects the illumination times for R, G, and B by multiplying the default or the illumination time information by a predetermined coefficient. Thus, even if the endoscope 2 has a plurality of CCDs 2a with different observation modes or even if the endoscope 2 with a different observation mode is connected, the amount of illumination light from the light source device 5 is optimized.

Note that the light source lamp 21 may decrease in brightness due to deterioration with age. In the case, the illumination time correcting portion of the utilization factor controlling portion 18 may be configured to correct the utilization factor depending on deterioration with age, i.e., an accumulated on-time of the light source lamp 21. With the configuration, the amount of light from the light source lamp 21 is controlled to be optimized depending on deterioration with age of the light source lamp 21.

### (Second Embodiment)

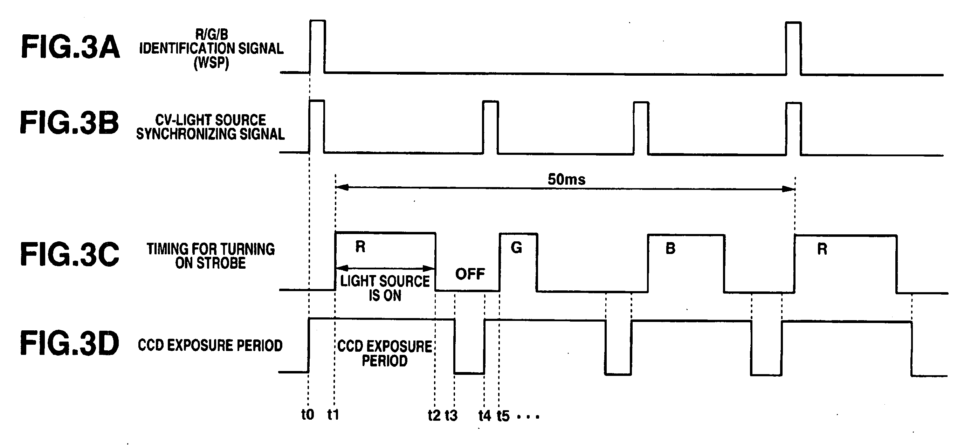

Figs. 3A to 3D relate to a second embodiment of the present invention. Fig. 3A is a chart showing timing for an R/G/B identification signal outputted from a utilization factor controlling portion of a light source device to a video signal processing portion in the second embodiment. Fig. 3B is a chart showing timing for a CV-light source synchronizing signal generated by a timing generator of a video processor in the second embodiment. Fig. 3C is a chart showing timing for turning on a stroboscopic light source in the second embodiment. Fig. 3D is a chart showing CCD exposure periods (image pickup periods) in the second embodiment. Note that same signals as in the first embodiment are denoted by same reference characters in Figs. 3A to 3D.

In an endoscope apparatus 1 according to the second embodiment, a light source portion 14 and an illumination time variator unit 15 of a light source device 5 have different configurations.

More specifically, the light source portion 14 is a stroboscopic light source which emits strobe light for a set time, and the illumination time variator unit 15 is configured to change illumination times for the light source device 5 by controlling light emission timing for the stroboscopic light source.

A utilization factor controlling portion 18 sets a utilization factor for the light source device 5 by controlling timing for turning on the stroboscopic light source for the illumination time variator unit 15 such that the illumination times for the light source device 5 are consistent with loaded illumination time information, if an endoscope 2 having a storage portion 8 is connected.

In the case, the utilization factor controlling portion 18 controls the timing for turning on the stroboscopic light source for the illumination time variator unit 15 such that an illumination time from t1 to t2 when an R transmission portion of an RGB rotating filter plate 24 is located in an optical path is a period based on the illumination time information. After that, the utilization factor controlling portion 18 similarly controls an illumination time for each of a G transmission portion and a B transmission portion of the rotating filter plate 24.

For a period for switching from the R transmission portion of the rotating filter plate to the G transmission portion in the optical path (a period from t2 to t5 in Fig. 3C), the utilization factor controlling portion 18 provides control to turn off the stroboscopic light source. After that, the utilization factor controlling portion 18 similarly provides control for a period for switching from the G transmission portion of the rotating filter plate 24 and for a period for switching from the B transmission portion.

Note that the timing for turning on the stroboscopic light source needs to be set such that the stroboscopic light source is on during a CCD exposure period (image pickup period) shown in Fig. 3D, as in the invention. The endoscope apparatus 1 according to the second embodiment need not be of a frame sequential type and can be applied to a simultaneous type endoscope apparatus.

According to the second embodiment, even if the illumination time variator unit 15 is constructed using a stroboscopic light source, same advantages as in the first embodiment can be achieved.

Note that the present invention is not limited to the above-described embodiments and that various modifications and applications can, of course, be made without departing from the invention as defined in the claims.

## Claims

1. An endoscope apparatus (1) comprising:
a connection portion (6a) adapted to be connected to medical equipment (2) having an image pickup portion (2a) having an exposure time range;
a light source portion (14) adapted to apply illumination light to an object to be observed by the image pickup portion (2a);
**characterized by** further comprising
an illumination time changing portion (15) adapted to change an illumination time that illumination light from the light source portion (14) is applied to the object to be observed;
a storage portion (8) provided in the medical equipment (2) adapted to store illumination time information for illumination light suitable for the medical equipment (2); and
a control portion (16, 17, 18, 19) adapted to read out the illumination time information stored in the storage portion (8) upon connection of the medical equipment (2) and adapted to control the illumination time changing portion (15) such that each illumination time that the light source portion (14) applies the illuminating light to the object to be observed by the image pickup portion (2a) is consistent with the read-out illumination time information.

2. The endoscope apparatus according to claim 1, wherein
if the control portion (16, 17, 18, 19) cannot read out the illumination time information from the storage portion (8), the control portion (16, 17, 18, 19) is adapted to determine a type of a CCD of the image pickup portion (2a) in the connected medical equipment and is adapted to control the illumination time changing portion (15) to change the illumination time for the light source portion (14) based on a determination result.

3. The endoscope apparatus according to claim 1, wherein
the control portion (16, 17, 18, 19) is adapted to determine a type of a lamp used as the light source portion (14) and is adapted to control the illumination time changing portion (15) to change the illumination time for the light source portion (14) based on a determination result.

4. The endoscope apparatus according to claim 1, wherein
the medical equipment is an endoscope (1) capable of switching between a plurality of observation modes, and the control portion (16, 17, 18, 198) is adapted to change the illumination time for the light source portion (14) upon switching between the observation modes in the endoscope (1).

5. The endoscope apparatus according to claim 1, wherein
the illumination time changing portion (15) is adapted to change the illumination time for the light source portion (14) by controlling a rotational phase difference between a plurality of rotating filters (24) arranged in an optical path of the light source portion (14).

6. The endoscope apparatus according to claim 1, wherein
the light source portion (14) is a stroboscopic light source adapted to emit strobe light for a set time, and
the illumination time changing portion (15) is adapted to change the illumination time for the light source portion (14) by controlling light emission timing for the stroboscopic light source.

7. The endoscope apparatus according to claim 1, wherein
the storage portion (8) is provided in a light source device having the light source portion (14).

8. The endoscope apparatus according to claim 1, wherein
a plurality of the image pickup portions (2a) are provided in the medical equipment (2), and
the storage portion (8) is adapted to store illumination time information corresponding to each of observation modes for the plurality of the image pickup portions (2a).

9. The endoscope apparatus according to claim 1,
wherein the illumination time information comprises illumination time information corresponding to respective illumination times for R light, G light, and B light, and
wherein the control portion (16 to 19) controls the illumination time changing portion (15) to change illumination times for which each of the R light, G light, and B light are applied to the object to be observed by the image pickup portion (2a) according to the respective illumination times for the R light, G light, and B light of the illumination time information.

## Patentansprüche

1. Endoskopvorrichtung (1) mit:
einem Anschlussbereich (6a), der dazu ausgebildet ist, mit einer Medizineinrichtung (2) verbunden zu werden, die einen Bildaufnahmebereich (2a) mit einer Belichtungszeit-Bandbreite aufweist;
einem Lichtquellenbereich (14), der bei einem durch den Bildaufnahmebereich (2a) zu beobachtenden Patienten Beleuchtungslicht anzuwenden vermag;
**dadurch gekennzeichnet, dass** sie des Weiteren aufweist:
einen Beleuchtungszeit-Änderungsbereich (15), der eine Beleuchtungszeit zu ändern vermag, in der Beleuchtungslicht vom Lichtquellenbereich (14) bei dem zu beobachtenden Patienten angewendet wird;
einen in der Medizineinrichtung (2) vorgesehenen Speicherbereich (8), der Beleuchtungszeitinformationen über das für die Medizineinrichtung (2) geeignete Beleuchtungslicht zu speichern vermag; und
einen Steuerungsbereich (16, 17, 18, 19), der nach Anschluss der Medizineinrichtung (2) die im Speicherbereich (8) gespeicherten Beleuchtungszeitinformationen auszulesen vermag und den Beleuchtungszeit-Änderungsbereich (15) so zu steuern vermag, dass jede Beleuchtungszeit, in welcher der Lichtquellenbereich (14) das Beleuchtungslicht beim durch den Bildaufnahmebereich (2a) zu beobachtenden Patienten anwendet, mit den ausgelesenen Beleuchtungszeitinformationen übereinstimmt.

2. Endoskopvorrichtung nach Anspruch 1, wobei
wenn der Steuerungsbereich (16, 17, 18, 19) die Beleuchtungszeitinformationen nicht aus dem Speicherbereich (8) auslesen kann, der Steuerungsbereich (16, 17, 18, 19) einen CCD-Typ des Bildaufnahmebereichs (2a) in der angeschlossenen Medizineinrichtung zu ermitteln vermag und den Beleuchtungszeit-Änderungsbereich (15) so zu steuern vermag, dass sich die Beleuchtungszeit für den Lichtquellenbereich (14) auf der Grundlage eines Ermittlungsergebnisses ändert.

3. Endoskopvorrichtung nach Anspruch 1, wobei
der Steuerungsbereich (16, 17, 18, 19) einen als Lichtquellenbereich (14) benutzten Lampentyp zu ermitteln vermag und den Beleuchtungszeit-Änderungsbereich (15) so zu steuern vermag, dass sich die Beleuchtungszeit für den Lichtquellenbereich (14) auf der Grundlage eines Ermittlungsergebnisses ändert.

4. Endoskopvorrichtung nach Anspruch 1, wobei
die Medizineinrichtung ein Endoskop (1) ist, das dazu ausgebildet ist, zwischen einer Vielzahl von Beobachtungsmodi zu schalten, und der Steuerungsbereich (16, 17, 18, 19) die Beleuchtungszeit für den Lichtquellenbereich (14) beim Schalten zwischen den Beobachtungsmodi im Endoskop (1) zu ändern vermag.

5. Endoskopvorrichtung nach Anspruch 1, wobei
der Beleuchtungszeit-Änderungsbereich (15) die Beleuchtungszeit für den Lichtquellenbereich (14) durch Steuern einer Drehphasendifferenz zwischen einer Vielzahl von Drehfiltern (24), die auf einem optischen Pfad des Lichtquellenbereichs (14) angeordnet sind, zu ändern vermag.

6. Endoskopvorrichtung nach Anspruch 1, wobei
der Lichtquellenbereich (14) eine Stroboskoplichtquelle ist, die über eine eingestellte Zeit stroboskopisches Licht auszusenden vermag, und
der Beleuchtungszeit-Änderungsbereich (15) die Beleuchtungszeit für den Lichtquellenbereich (14) durch Steuern des Lichtemissionstakts für die Stroboskoplichtquelle zu ändern vermag.

7. Endoskopvorrichtung nach Anspruch 1, wobei
der Speicherbereich (8) in einer Lichtquelleneinrichtung mit dem Lichtquellenbereich (14) vorgesehen ist.

8. Endoskopvorrichtung nach Anspruch 1, wobei
eine Vielzahl von Bildaufnahmebereichen (2a) in der Medizineinrichtung (2) vorgesehen ist, und
der Speicherbereich (8) die Beleuchtungszeitinformationen entsprechend jedem der Beobachtungsmodi für die Vielzahl der Bildaufnahmebereiche (2a) zu speichern vermag.

9. Endoskopvorrichtung nach Anspruch 1,
wobei die Beleuchtungszeitinformationen Beleuchtungszeitinformationen entsprechend den jeweiligen Beleuchtungszeiten für R-Licht, G-Licht und B-Licht aufweisen, und
wobei der Steuerungsbereich (16 bis 19) den Beleuchtungszeit-Änderungsbereich (15) so steuert, dass er die Beleuchtungszeiten, während denen jede der Lichtarten R-Licht, G-Licht und B-Licht bei dem durch den Bildaufnahmebereich (2a) zu beobachtenden Patienten angewendet werden, entsprechend den jeweiligen Beleuchtungszeiten für R-Licht, G-Licht und B-Licht Beleuchtungszeitinformationenen ändert.

## Revendications

1. Appareil d'endoscope (1) comprenant :
une partie de connexion (6a) adaptée pour être connectée à un équipement médical (2) comportant une partie de capture d'images (2a) ayant une plage de temps d'exposition ;
une partie de source de lumière (14) adaptée pour appliquer une lumière d'éclairage sur un objet devant être observé par la partie de capture d'images (2a) ;
**caractérisé en ce qu'**il comprend de plus
une partie de changement de temps d'éclairage (15) adaptée pour changer un temps d'éclairage pendant lequel la lumière d'éclairage provenant de la partie de source de lumière (14) est appliquée sur l'objet devant être observé ;
une partie de mémoire (8) prévue dans l'équipement médical (2) adaptée pour mémoriser une information de temps d'éclairage pour une lumière d'éclairage appropriée pour l'équipement médical (2) ; et
une partie de commande (16, 17, 18, 19) adaptée pour extraire l'information de temps d'éclairage mémorisée dans la partie de mémoire (8) sur connexion de l'équipement médical (2) et adaptée pour commander la partie de changement de temps d'éclairage (15) d'une manière telle que chaque temps d'éclairage pendant lequel la partie de source de lumière (14) applique la lumière d'éclairage sur l'objet devant être observé par la partie de capture d'images (2a) est en accord avec l'information de temps d'éclairage extraite.

2. Appareil d'endoscope selon la revendication 1, dans lequel
si la partie de commande (16, 17, 18, 19) ne peut pas extraire l'information de temps d'éclairage de la partie de mémoire (8), la partie de commande (16, 17, 18, 19) est adaptée pour déterminer un type de CCD de la partie de capture d'images (2a) dans l'équipement médical connecté et est adaptée pour commander la partie de changement de temps d'éclairage (15) pour changer le temps d'éclairage de la partie de source de lumière (14) sur la base d'un résultat de détermination.

3. Appareil d'endoscope selon la revendication 1, dans lequel
la partie de commande (16, 17, 18, 19) est adaptée pour déterminer un type de lampe utilisée en tant que partie de source de lumière (14) et est adaptée pour commander la partie de changement de temps d'éclairage (15) pour changer le temps d'éclairage de la partie de source de lumière (14) sur la base d'un résultat de détermination.

4. Appareil d'endoscope selon la revendication 1, dans lequel
l'équipement médical est un endoscope (1) capable de commuter entre une pluralité de modes d'observation, et la partie de commande (16, 17, 18, 19) est adaptée pour changer le temps d'éclairage de la partie de source de lumière (14) sur commutation entre les modes d'observation dans l'endoscope (1).

5. Appareil d'endoscope selon la revendication 1, dans lequel
la partie de changement de temps d'éclairage (15) est adaptée pour changer le temps d'éclairage de la partie de source de lumière (14) en commandant une différence de phase de rotation entre une pluralité de filtres rotatifs (24) agencés dans un trajet optique de la partie de source de lumière (14).

6. Appareil d'endoscope selon la revendication 1, dans lequel
la partie de source de lumière (14) est une source de lumière stroboscopique adaptée pour émettre une lumière stroboscopique pendant un temps établi, et
la partie de changement de temps d'éclairage (15) est adaptée pour changer le temps d'éclairage de la partie de source de lumière (14) en commandant la cadence d'émission de lumière de la source de lumière stroboscopique.

7. Appareil d'endoscope selon la revendication 1, dans lequel
la partie de mémoire (8) est prévue dans le dispositif de source de lumière comportant la partie de source de lumière (14).

8. Appareil d'endoscope selon la revendication 1, dans lequel
une pluralité de parties de capture d'images (2a) sont prévues dans l'équipement médical (2), et
la partie de mémoire (8) est adaptée pour mémoriser une information de temps d'éclairage correspondant à chacun des modes d'observation pour la pluralité de parties de capture d'images (2a).

9. Appareil d'endoscope selon la revendication 1,
dans lequel l'information de temps d'éclairage comprend une information de temps d'éclairage correspondant aux temps d'éclairage respectifs pour une lumière rouge, une lumière verte et une lumière bleue, et
dans lequel la partie de commande (16 à 19) commande la partie de changement de temps d'éclairage (15) pour changer les temps d'éclairage pendant lesquels chacune parmi la lumière rouge, la lumière verte et la lumière bleue est appliquée à l'objet devant être observé par la partie de capture d'images (2a) conformément aux temps d'éclairage respectifs pour la lumière rouge, la lumière verte et la lumière bleue de l'information de temps d'éclairage.
